# EUROPEAN PATENT APPLICATION

(11) **EP 4 316 475 A1**
(43) Date of publication of application: **07.02.2024**
(21) Application number: 22775501.4
(22) Date of filing: 18.03.2022
(51) Int. Cl.: A61K 31/045, A61K 8/34, A61P 17/00, A61P 25/00, A61P 25/02, A61P 43/00, A61Q 19/00, C12N 5/071, C12Q 1/02, G01N 33/15, G01N 33/483, G01N 33/50

(54) **MERKEL CELL ACTIVATOR, SYNAPTIC VESICLE ENHANCER, NEUROTRANSMITTER RELEASE ACCELERATOR, METHOD FOR ASSESSING NEUROTRANSMITTER RELEASE ACTIVITY OF MERKEL CELLS, AND METHOD FOR SCREENING FOR NEUROTRANSMITTER RELEASE ACCELERATOR FOR MERKEL CELLS**

(30) Priority: 22.03.2021 JP 2021047727; 02.06.2021 JP 2021093246
(71) Applicant: Shiseido Company, Ltd., Chuo-ku Tokyo 104-0061 (JP)
(72) Inventor: TOJYO, Moe, Tokyo 104-0061 (JP); KAJIYA, Kentaro, Tokyo 104-0061 (JP); SAKAGUCHI, Saito, Tokyo 104-0061 (JP)
(74) Representative: Cabinet Beau de Loménie
(86) International application number: PCT/JP2022/012849
(87) International publication number: WO 2022/202714

(57) **Abstract**

The purpose of the present invention is to provide skin care focusing on the sense of touch. It was found that sandalore and an analog thereof have an action of activating Merkel cells. Provided is A Merkel cell activating agent containing sandalore and an analog thereof. It was also found that an activated Merkel cell contains a large number of synaptic vesicles. Provided is a synaptic vesicle enhancing agent containing sandalore and an analog thereof. It was also found that the neurotransmitter release activity of Merkel cells can be assessed by measuring, under a fluorescence microscope, changes in fluorescent luminance value before and after depolarization stimulation to Merkel cells that have taken in a fluorescent tracer. Provided is a method for screening for a neurotransmitter release accelerating agent for Merkel cells.

## Description

### FIELD

The present invention relates to a Merkel cell activating agent, and to a synaptic vesicle enhancing agent for Merkel cells.

The present invention further relates to a method for assessing neurotransmitter release activity of Merkel cells, to a method for screening for a neurotransmitter release accelerating agent utilizing the assessment method, and to a neurotransmitter release accelerating agent screened by the screening method.

### BACKGROUND

Receptors associated with tactile sensation are excited by mechanical stimulation, and as a result thereof, a signal is sent to the brain via sensory nerves. Examples of receptors excited by mechanical stimuli include Merkel cells present in the deepest regions of the epidermis, Meissner's corpuscles present in the dermis outermost layer, and Pacinian corpuscles and Ruffini endings found in the deep dermis. Sensory nerve endings are connected through synapses to Merkel cells, neurotransmitters are secreted into the synapses when the cells are excited by mechanical stimuli, and information is transmitted to sensory nerves. It has been suggested that Merkel cell Piezo2 channels are involved in sensing of mechanical stimulation (NPL 1: Nature (2014) Column 509, pages 622-626). It has been reported that when Merkel cells are decreased, this can lead to alloknesis, a condition in which itching is induced by stimuli that normally do not produce itching sensation, while activation of Merkel cells inhibits itching (NPL 5). It has also been reported that PIEZO2-deficient individuals are unable to induce sensitization or pain reaction even at sites of skin inflammation, and that PIEZO2 channels are therapeutic targets for allodynia, a condition in which pain is produced by non-noxious stimulation which normally does not cause pain (NPL 6).

On the other hand, OR2AT4, which is an olfactory receptor, is expressed in keratinocytes, the main constituent cells of the epidermis, and sandalore, which is an antagonist thereof, has been reported to promote wound healing by inducing intracellular signals by acting on OR2AT4 (NPL 2: J Investigative Dermatology (2014) vol. 134, p. 2823-2832). The receptor OR2AT4 is also expressed in hair follicle sheath cells, and application of Sandalore has been reported to induce hair extension (PTL 1: Japanese Patent Public Inspection No. 2019-519502, NPL 3: Nat Commun. (2018) Sep 18; 9(1):3624).

### [CITATION LIST]

### [PATENT LITERATURE]

[PTL 1] Japanese Patent Public Inspection No. 2019-519502

### [NON PATENT LITERATURE]

[NPL 1] Nature (2014)volume 509, p. 622-626
[NPL 2] Journal of Investigative Dermatology (2014) 134:2823-2832
[NPL 3] Nat Commun. (2018) Sep 18; 9(1):3624
[NPL 4] Neuron (2018)19:100(6):1401-1413
[NPL 5] Science. 2018 May 04; 360(6388): 530-533. doi:10.1126/science.aar570
[NPL 6] Sci Transl Med. (2018)10(462)eaat9892. doi: 10.1126/scitranslmed. aat9892

### SUMMARY

### [TECHNICAL PROBLEM]

The inventors of the present invention have conducted extensive research on skin care with focus on tactile sensation, and an object of the present invention is to obtain a drug that activates Merkel cells.

Activity by Merkel cells has conventionally been evaluated using changes in the levels of certain proteins expressed by the Merkel cells as the index. However, the amount of such proteins vary over relatively long periods of several hours to several days, making it impossible to evaluate immediate changes in activity. The amount of such proteins also merely enables for indirect measurement of Merkel cell activity. It is an object of the present invention to measure direct and immediate activity of Merkel cells during depolarization.

### [SOLUTION TO PROBLEM]

When the inventors of the present invention focused on OR2AT4, which is an olfactory receptor expressed in the skin, and examined the presence thereof in cell lines other than keratinocytes, it was surprisingly found that OR2AT4 is expressed in Merkel cells. Moreover, it was also found that Merkel cells are activated by applying the OR2AT4 agonist, sandalore, to Merkel cells, thereby leading to completion of the present invention.

The present invention relates to the following:
[A1-1] A Merkel cell activating agent, comprising a compound represented by the following formula: 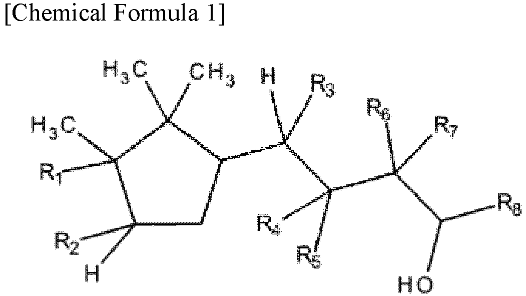 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A1-2] A compound represented by the following formula, for use in treatment or prevention of at least one symptom selected from the group consisting of itching, skin pruritus, alloknesis and allodynia through activation of Merkel cells: 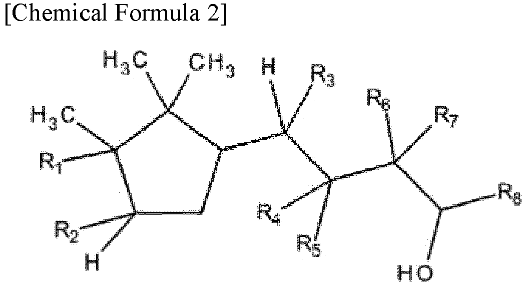 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A1-3] A cosmetic method through activation of Merkel cells, comprising administering a compound represented by the following formula: 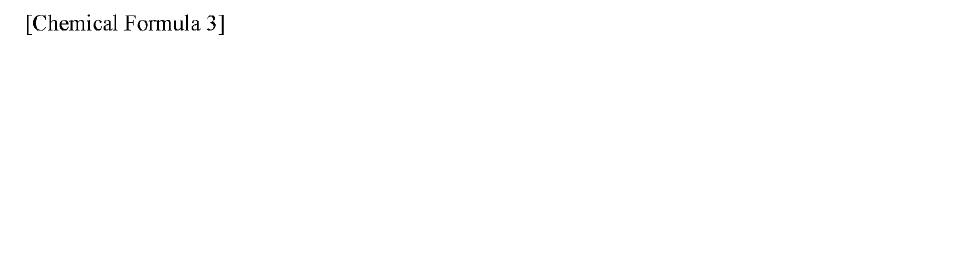 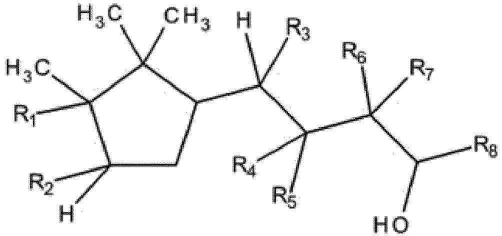 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A1-4] A method for activation of Merkel cells, comprising administering a compound represented by the following formula to a subject in need of activation of Merkel cells: 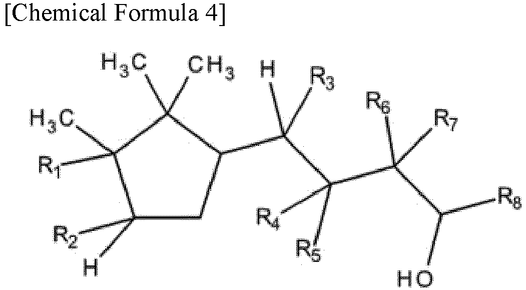 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A1-5] Use of a compound represented by the following formula for production of a Merkel cell activating agent: 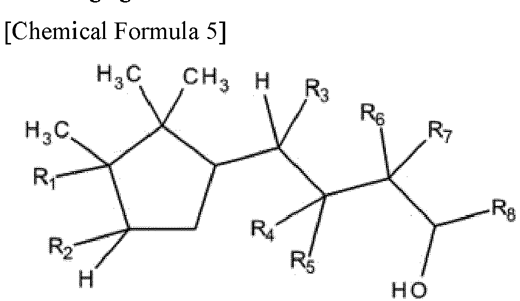 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A2] The activating agent, compound, cosmetic method, method for activation or use according to any one of [A1-1] to [A1-5] above, wherein the compound is Sandalore or Brahmanol.
[A3] The activating agent, compound, cosmetic method, method for activation or use according to any one of [A1-1] to [A1-5] or [A2] above, which enhances tactile sensation.
[A3-2] The activating agent, compound, cosmetic method, method for activation or use according to any one of [A1-1] to [A1-5], [A2] or [A3] above, which is for treatment, prevention or alleviation of at least one symptom selected from the group consisting of itching, skin pruritus, alloknesis and allodynia.
[A4-1] A synaptic vesicle enhancing agent for Merkel cells, comprising a compound represented by the following formula: 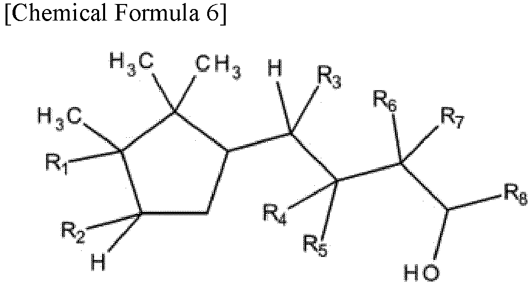 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A4-2] A compound represented by the following formula, for use in treatment, prevention or alleviation of at least one symptom selected from the group consisting of itching, skin pruritus, alloknesis and allodynia via enhancement of synaptic vesicles in Merkel cells: 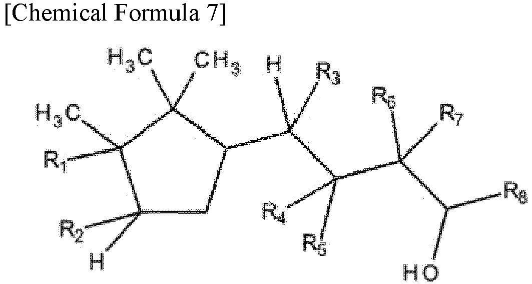 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A4-3] A cosmetic method through enhancement of synaptic vesicles in Merkel cells, comprising administering a compound represented by the following formula: 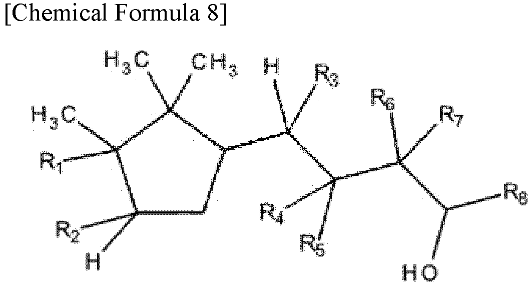 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   Rg is hydrogen or methyl].
[A4-4] An enhancement method for synaptic vesicles in Merkel cells, comprising administering a compound represented by the following formula to a subject in need of enhancement of synaptic vesicles in Merkel cells: 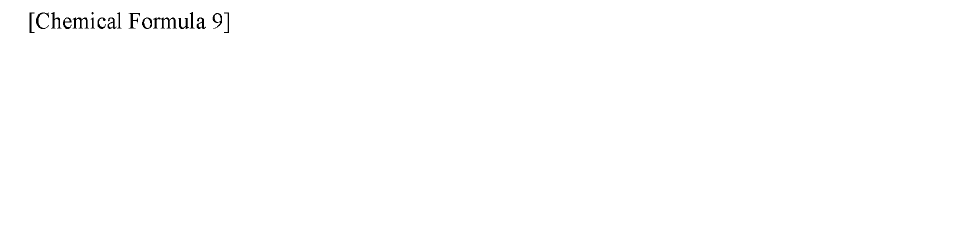 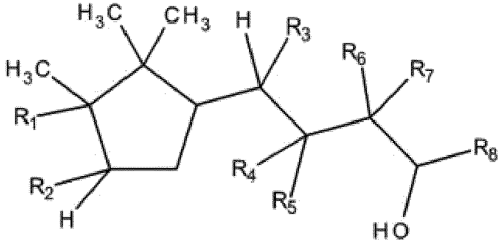 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A4-5] Use of a compound represented by the following formula for production of a synaptic vesicle enhancing agent for Merkel cells: 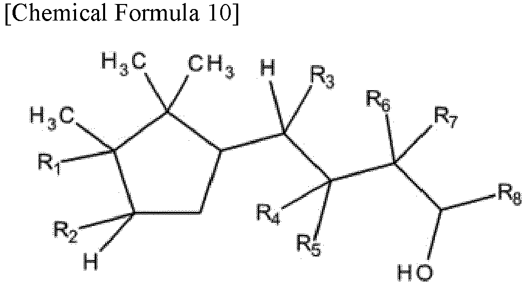 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[A5] The activating agent, compound, cosmetic method, method for activation or use according to any one of [A4-1] to [A4-5] above, wherein the compound is Sandalore or Brahmanol.
[A6] The activating agent, compound, cosmetic method, method for activation or use according to any one of [A4-1] to [A4-5] or [A5] above, wherein the enhancing agent increases the efficiency of neurotransmission between Merkel cells and neurons.
[A7] The activating agent, compound, cosmetic method, method for activation or use according to any one of [A4-1] to [A4-5], [A5] or [A6] above, which enhances tactile sensation.
[A8] The activating agent, compound, cosmetic method, method for activation or use according to any one of [A4-1] to [A4-5] or [A5] to [A7] above, which enhances collagen production.
[A9] The activating agent, compound, cosmetic method, method for activation or use according to any one of [A4-1] to [A4-5] or [A5] to [A8] above, which is for treatment, prevention or alleviation of at least one symptom selected from the group consisting of itching, skin pruritus, alloknesis and allodynia.

The present inventors further conducted avid research on the mechanism of release of neurotransmitters by excitation of Merkel cells, focusing on perceptible tactile sensation with skin care, for example, and as a result devised the present invention upon finding that with Merkel cells incorporating a fluorescent tracer, the neurotransmitter release activity of the Merkel cells can be evaluated by measuring the change in fluorescent brightness value before and after depolarizing stimulus under a fluorescent microscope.

The present invention relates to the following:
[B1] A method for assessing neurotransmitter release activity of Merkel cells in a skin sample, comprising:
   culturing the skin sample in medium containing a neurotransmitter fluorescent tracer;
   applying depolarizing stimulus; and
   measuring the change in fluorescent brightness value of the Merkel cells before and after depolarizing stimulus, under a fluorescent microscope.
[B2] The assessment method according to [B1] above, wherein the neurotransmitter fluorescent tracer is a monoamine neurotransmitter fluorescent tracer.
[B3] The assessment method according to [B1] or [B2], wherein the skin sample is a human skin sample.
[B4] A method for screening a neurotransmitter release accelerating agent for Merkel cells in a skin sample, wherein the method includes:
   culturing the skin sample in medium containing a neurotransmitter fluorescent tracer;
   adding a candidate substance for a neurotransmitter release accelerating agent; and
   measuring the change in fluorescent brightness value of the Merkel cells before and after addition, under a fluorescent microscope.
[B5] The screening method according to [B4] above, wherein the neurotransmitter fluorescent tracer is a monoamine neurotransmitter fluorescent tracer.
[B6] The screening method according to [B4] or [B5], wherein the skin sample is a human skin sample.
[B7-1] A neurotransmitter release accelerating agent for Merkel cells, comprising a compound represented by the following formula: 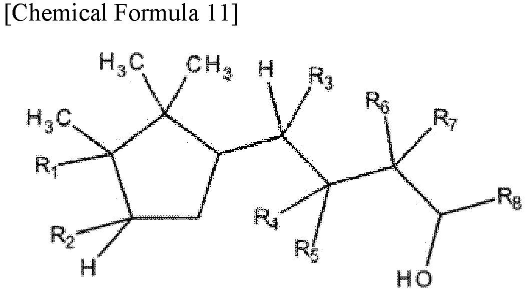 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   Rg is hydrogen or methyl].
[B7-2] A compound represented by the following formula for use in treatment or prevention of at least one symptom selected from the group consisting of itching, skin pruritus, alloknesis and allodynia via promoting release of neurotransmitters in Merkel cells: 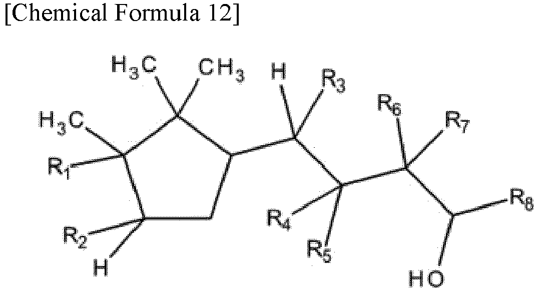 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   Rg is hydrogen or methyl].
[B7-3] A cosmetic method via promoting release of neurotransmitters in Merkel cells, comprising administering a compound represented by the following formula: 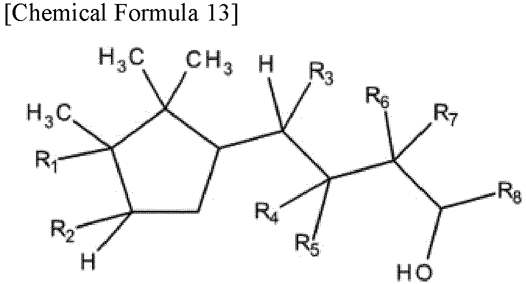 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[B7-4] A method for promoting release of neurotransmitters in Merkel cells, comprising administering a compound represented by the following formula to a subject in need of release of neurotransmitters in Merkel cells: 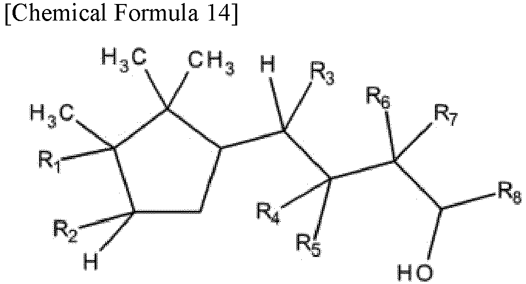 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[B7-5] Use of a compound represented by the following formula for production of a neurotransmitter release accelerating agent for Merkel cells: 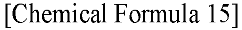 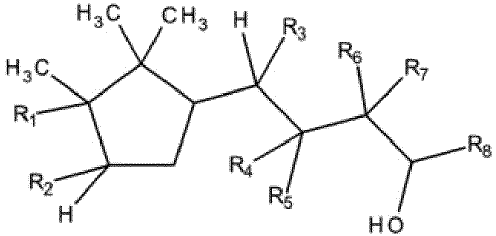 [wherein
   R₁ and R₂ together form a double bond, or R₁ and R₂ together form a cyclopropyl group;
   R₃ and R₄ are the same or different and are independently selected from hydrogen and methyl, or R₃ and R₄ together form a double bond;
   R₅ and R₆ are the same or different and are independently selected from hydrogen and methyl, or R₅ and R₆ together form a double bond; or R₅ and R₆ together form a cyclopropyl group;
   R₇ is methyl or ethyl; and
   R₈ is hydrogen or methyl].
[B8] The neurotransmitter release accelerating agent, compound, cosmetic method, promoting method or use according to any one of [B7-1] to [B7-5] above, wherein the compound is Sandalore or Brahmanol.
[B9] The neurotransmitter release accelerating agent, compound, cosmetic method, promoting method or use according to any one of [B7-1] to [B7-5] or [B8] above, which enhances tactile sensation.
[B10] The neurotransmitter release accelerating agent, compound, cosmetic method, promoting method or use according to any one of [B7-1] to [B7-5], [B8] or [B9] above, which enhances collagen production.
[B11] The neurotransmitter release accelerating agent, compound, cosmetic method, enhancement method or use according to any one of [B7-1] to [B7-5], [B8], [B9] or [B10] above, which is for treatment, prevention or alleviation of at least one symptom selected from the group consisting of itching, skin pruritus, alloknesis and allodynia.

### [ADVANTAGEOUS EFFECTS OF INVENTION]

Merkel cells can be activated by applying Sandalore and/or its analog. It is thus possible to provide a skin care method focusing on tactile sensation, via activation of Merkel cells.

It is also possible to evaluate neurotransmitter release activity when Merkel cells have been depolarized. The assessment method can be utilized for screening of a neurotransmitter release activity accelerating agent. Sandalore and its analogs were also screened as neurotransmitter release activity accelerating agents.

### BRIEF DESCRIPTION OF DRAWINGS

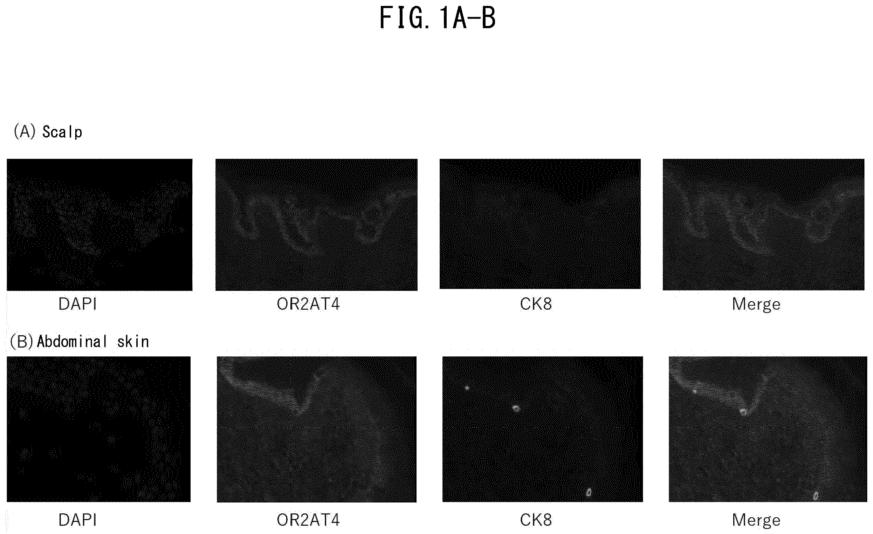

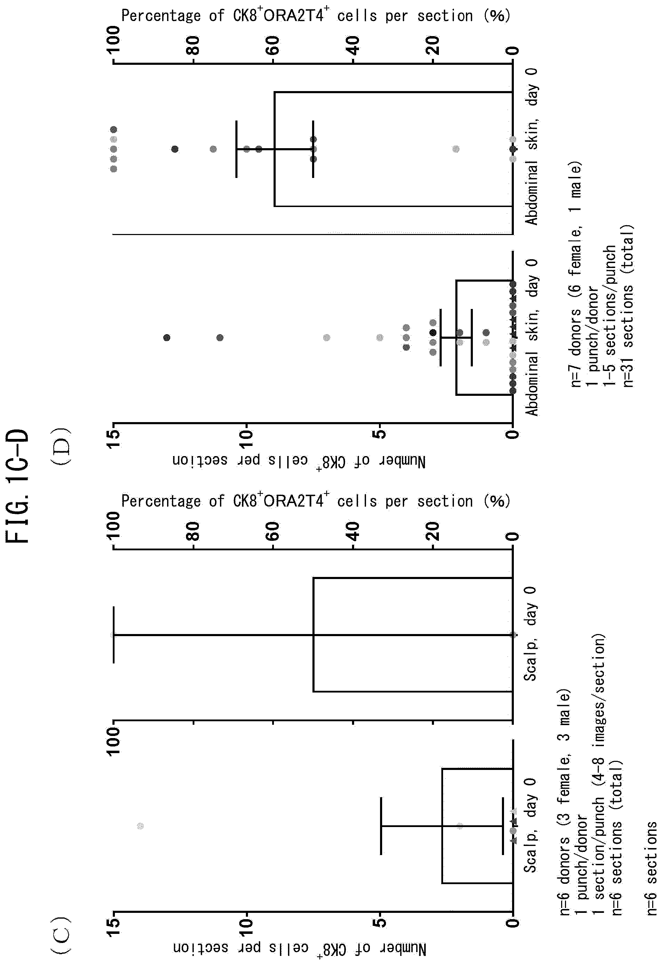

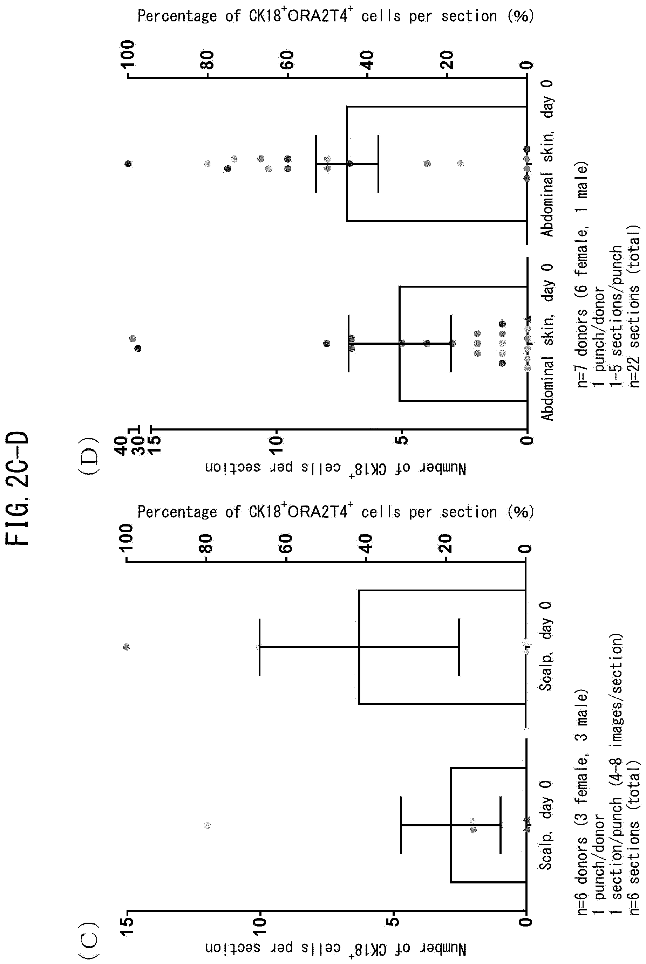

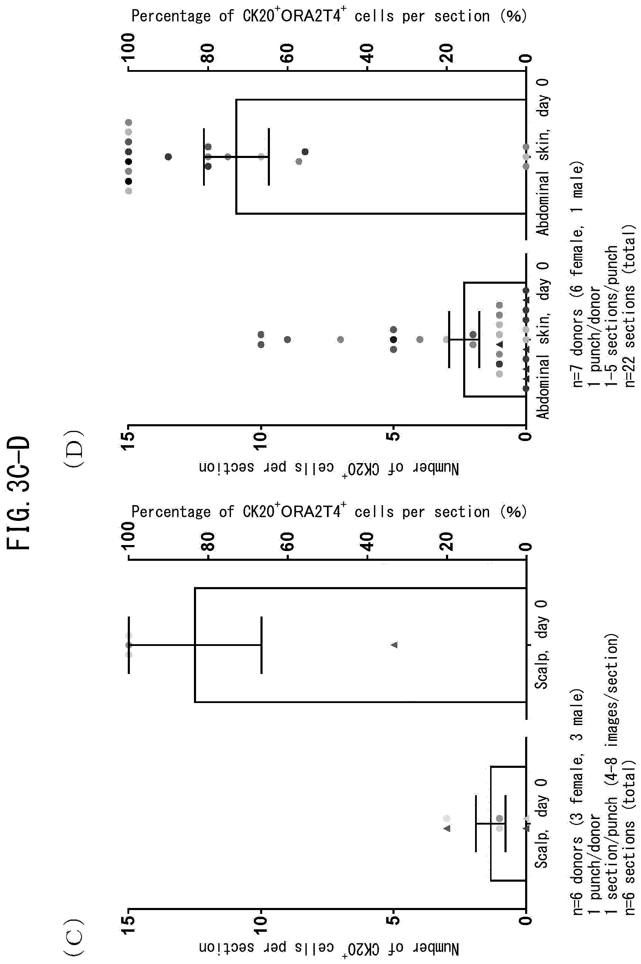

Figs. 1A and 1B show the results of co-staining a skin sample with the Merkel cell markers CK8 and OR2AT4, and observation under a fluorescent microscope. Scalp samples (A) and abdominal skin samples (B) were co-stained with CK8 and OR2AT4 and photographed.
The left graph in Fig. 1C shows the count of cells double-positive for OR2AT4 and CK8 in a specified region of the scalp sample. The right graph shows the ratio of cells double-positive for OR2AT4 and CK8 with respect to CK8-positive cells in the scalp sample. The left graph in Fig. 1D shows the count of cells double-positive for OR2AT4 and CK8 in a specified region of the abdominal sample. The right graph shows the ratio of cells double-positive for OR2AT4 and CK8 with respect to CK8-positive cells in the abdominal sample.

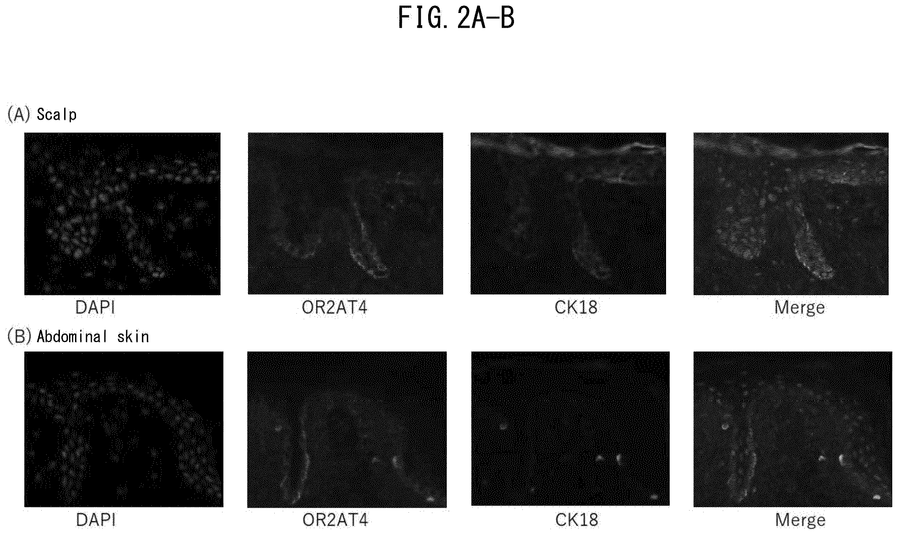

Figs. 2A and 2B show the results of co-staining a skin sample with the Merkel cell markers CK18 and OR2AT4, and observation under a fluorescent microscope. Scalp samples (A) and abdominal skin samples (B) were co-stained with CK18 and OR2AT4 and photographed.
The left graph in Fig. 2C shows the count of cells double-positive for OR2AT4 and CK18 in a specified region of the scalp sample. The right graph shows the ratio of cells double-positive for OR2AT4 and CK18 with respect to CK18-positive cells in the scalp sample. The left graph in Fig. 2D shows the count of cells double-positive for OR2AT4 and CK18 in a specified region of the abdominal sample. The right graph shows the ratio of cells double-positive for OR2AT4 and CK18 with respect to CK18-positive cells in the abdominal sample.

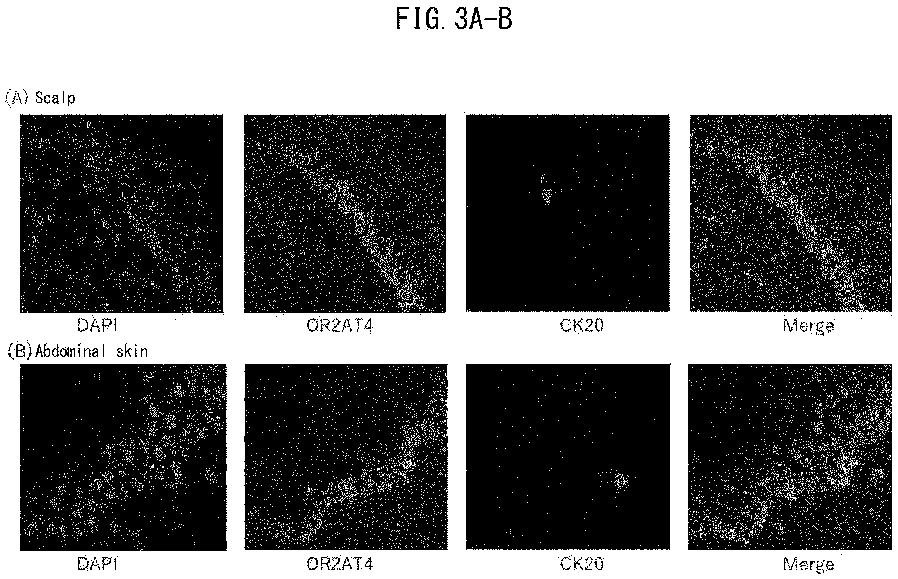

Figs. 3A and 3B show the results of co-staining a skin sample with the Merkel cell markers CK20 and OR2AT4, and observation under a fluorescent microscope. Scalp samples (A) and abdominal skin samples (B) were co-stained with CK20 and OR2AT4 and photographed.
The left graph in Fig. 3C shows the count of cells double-positive for OR2AT4 and CK20 in a specified region of the scalp sample. The right graph shows the ratio of cells double-positive for OR2AT4 and CK20 with respect to CK20-positive cells in the scalp sample. The left graph in Fig. 3D shows the count of cells double-positive for OR2AT4 and CK20 in a specified region of the abdominal sample. The right graph shows the ratio of cells double-positive for OR2AT4 and CK20 with respect to CK20-positive cells in the abdominal sample.

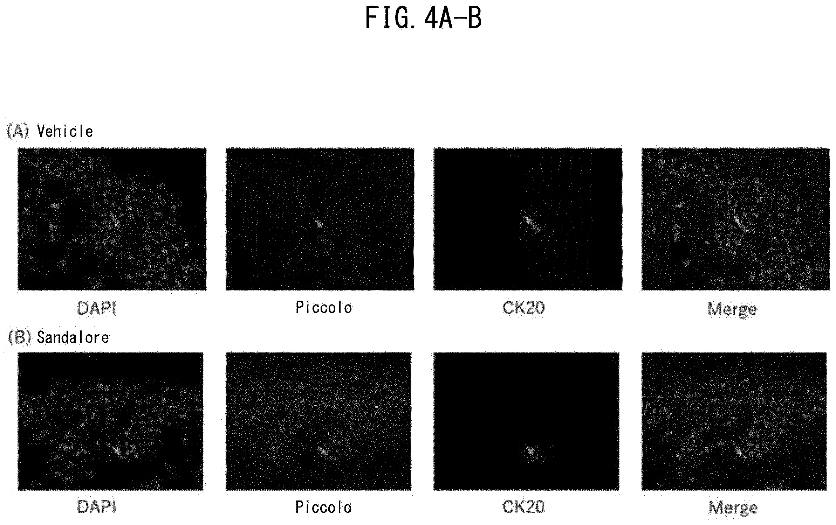

Figs. 4A and 4B show CK20-positive cells expressing Piccolo in a sample to which Sandalore was applied, photographed under a fluorescent microscope (Fig. 4A: control (vehicle), Fig. 4B: Sandalore).

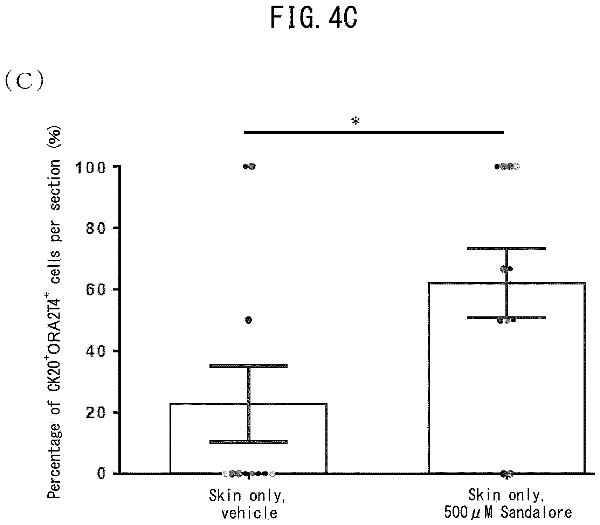

Fig. 4C is a graph showing the number of CK20-positive cells expressing Piccolo in a specified region in a photograph taken under a fluorescent microscope.

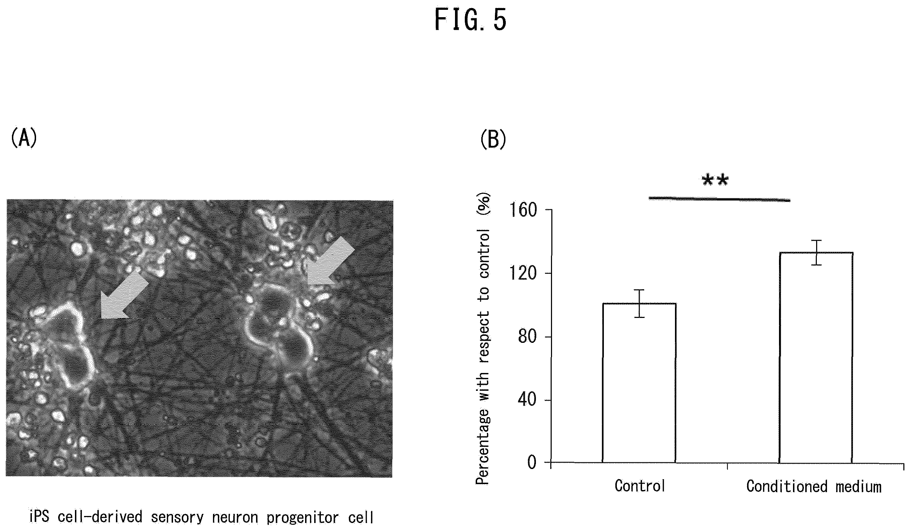

Fig. 5A shows iPS cell-derived sensory neuron progenitor cells. Fig. 5B shows the relative value for type I collagen mRNA expression level with addition of culture supernatant of iPS cell-derived sensory neuron progenitor cells (conditioned medium) to fibroblasts, compared to
   ** addition of unconditioned medium to fibroblasts. The error bars represent average ±SD and ** represents statistically significant difference in an unpaired t test (**P <0.01).

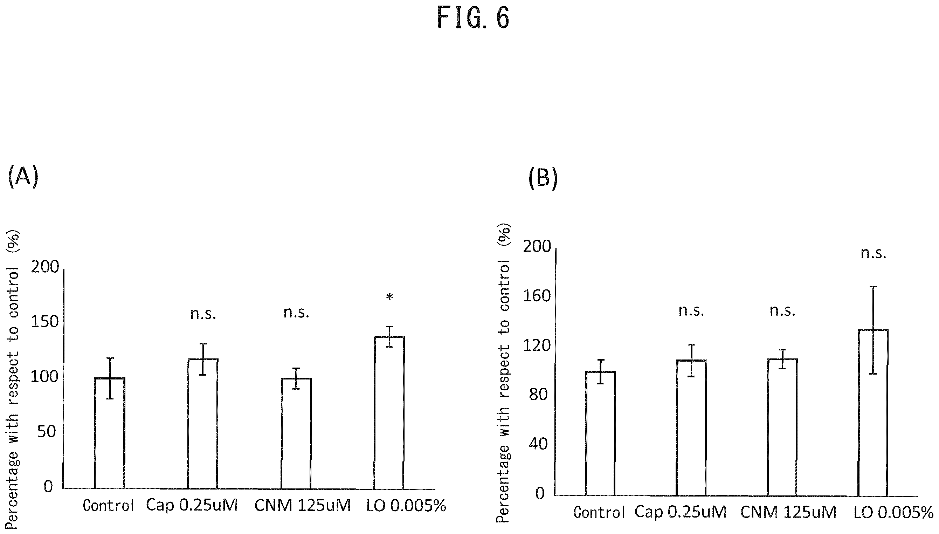

Fig. 6A shows mRNA expression level of type I collagen when medium supernatant of iPS cell-derived sensory neuron progenitor cells stimulated with a test substance (lavender oil: LO 0.005%) was added to fibroblasts. Fig. 6B shows mRNA expression level of type I collagen when the test substance was directly added to fibroblasts at the same concentration. In the graphs, collagen production is represented as a relative value with the control (test substance not added: control) as 100%. The error bars represent average ±SD, represents statistically significant difference in an unpaired t test (*P <0.05), and n.s. represents no statistically significant difference.

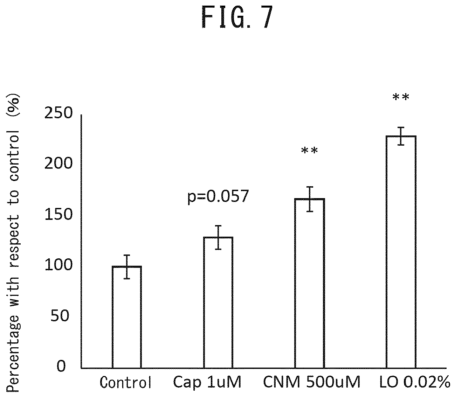

Fig. 7 shows mRNA expression level of type I collagen when a test substance at higher concentration than Fig. 6B (4-fold) (lavender oil: LO 0.02%) was added directly to fibroblasts. Collagen production is represented as a relative value with the control (test substance not added: ** control) as 100%. The error bars represent average ±SD,** represents statistically significant difference in an unpaired t test (**p <0.01), and n.s. represents no statistically significant difference.

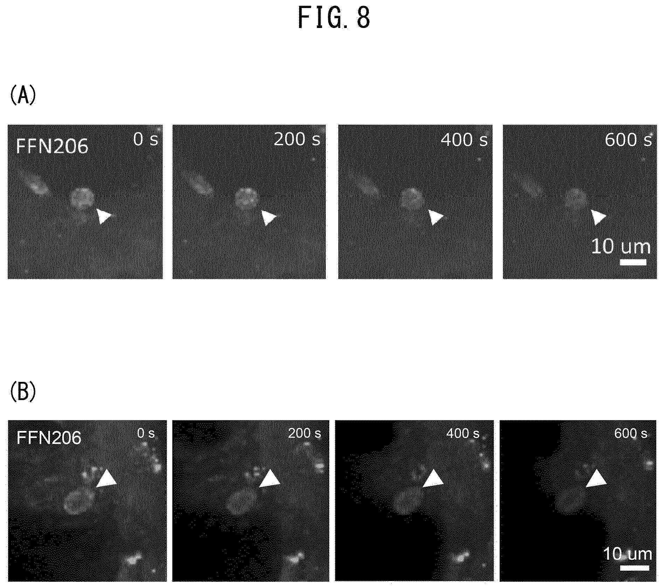

Fig. 8 is a set of photographs showing change in fluorescence in Merkel cells incorporating a fluorescent tracer (FFN206), after 0, 200, 400 and 600 seconds. Fig. 8A shows change in fluorescence after application of Sandalore stimulation from 300 seconds to 600 seconds, and Fig. 8B shows change in fluorescence after circulation of BSS solution from 300 seconds to 600 seconds, as a control.

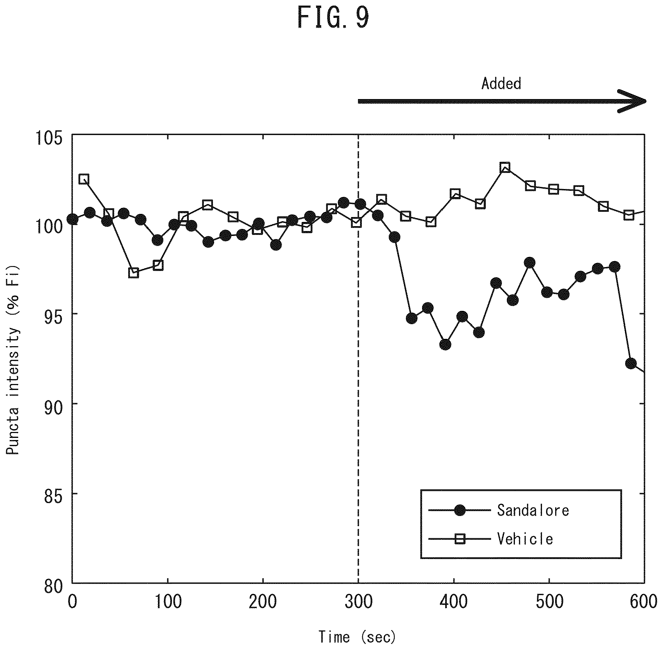

Fig. 9 is a graph showing change in fluorescent brightness value of Merkel cells in fluorescent imaging for 10 minutes. At 5 minutes (300 seconds), Sandalore was introduced and the fluorescent brightness value decreased (black circles). BSS solution was circulated at 5 minutes (300 seconds) as a control (white squares).

### DESCRIPTION OF EMBODIMENTS

The present invention relates to a Merkel cell activating agent comprising Sandalore (3-methyl-5-(2,2,3-trimethyl-cyclopenta-3-en-1-yl)-pentan-2-ol [CAS:65113-99-7]) or its analog. Sandalore is represented by the following chemical structure: 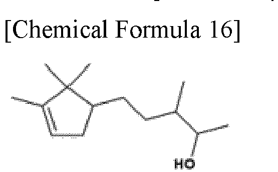 Sandalore was developed and marketed as a compound having the aroma of sandalwood. The aroma component of sandalwood is primarily santalol, including its stereoisomer. Santalol is known to have an α form and a β form. Santalol is difficult to synthesize due to its complex structure, and therefore aromatics having aroma similar to sandalwood but with simpler structures have been synthesized, with Sandalore being one of them. Sandalore acts as an agonist for the olfactory receptor OR2AT4. Sandalore and its analogs are compounds with agonistic action for OR2AT4. One example of a Sandalore analog with agonistic action for OR2AT4 is Brahmanol (2-methyl-4-(2,2,3-trimethyl-3 -cyclopenten-1 -yl)-butan-1 -ol [CAS:72089-08-8]) (NPL 2). Brahmanol is represented by the following chemical structure:

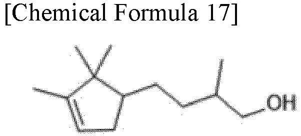

Examples of Sandalore or its analogs exhibiting agonistic action for OR2AT4 include the following compounds:

**[Table 1]**

| | IUPAC Nomenclature | CAS No. | Structural Formula | Formula/ MW |
|---|---|---|---|---|
| 1 | 3-methyl-5-(2,2,3-trimethylcyclopent-3-en-1-yl)pentan-2-ol | 65113-99-7 | 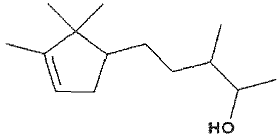 | C₁₄H₂₆O |
| | | | | 210.36 |
| 2 | (4Z)-3-methyl-5-(2,2,3-trimethyl cyclopent-3-en-1-yl)pent-4-en-2-ol | 67801-20-1 | 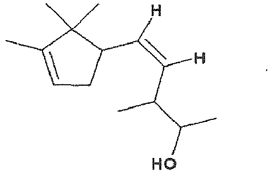 | C₁₄H₂₄O |
| | | | | 208.35 |
| 3 | 1-methyl-2-((1,2,2-trimethylbicyclo (3.1.0)hex-3-yl)methyl)-cyclopropane-methanol | 198404-98-7 | 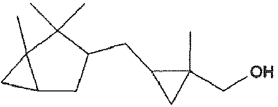 | C₁₅H₂₆O |
| | | | | 222.37 |
| 4 | (E)-3,3-dimethyl-5-(2,2,3-trimethyl cyclopent-3-en-1-yl)pent-4-en-2-ol | 107898-54-4 | 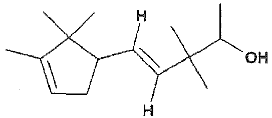 | C₁₅H₂₆O |
| | | | | 222.37 |
| 5 | 2-methyl-4-(2,2,3-trimethyl-3-cyclopenten-1-yl)butanol | 72089-08-8 | 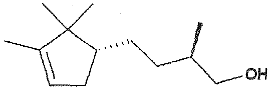 | C₁₃H₂₄O |
| | | | | 196.34 |
| 6 | (E)-2-ethyl-4-(2,2,3-trimethyl cyclopent-3-en-1-yl)but-2-en-1-ol | 28219-61-6 | 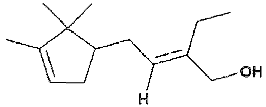 | C₁₄H₂₄O |
| | | | | 208.35 |
| 7 | (E)-2-methyl-4-(2,2,3)-trimethyl-3- cyclopenten-1-yl)-2-buten-1-ol | 28219-60-5 | 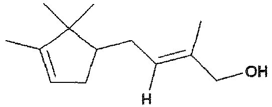 | C₁₃H₂₂O |
| | | | | 194.32 |

Examples of Sandalore or analogues thereof relate to compounds represented by the following formula: 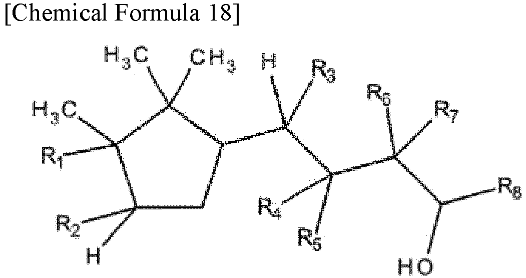 [wherein
R₁ and R₂ together form a double bond, or R₁ and R₂ together form a cyclopropyl group;
R₃ and R₄ are the same or different and are independently selected from hydrogen and methyl, or R₃ and R₄ together form a double bond;
R₅ and R₆ are the same or different and are independently selected from hydrogen and methyl, or R₅ and R₆ together form a double bond; or R₅ and R₆ together form a cyclopropyl group;
R₇ is methyl or ethyl; and
Rg is hydrogen or methyl]. These compounds may also include optical isomers. The compounds function as OR2AT4 agonists, similar to Sandalore. The compounds also activate Merkel cells, similar to Sandalore, and accelerate release of neurotransmitters from Merkel cells.

Activation of Merkel cells refers to proliferation or maturation of Merkel cells. Merkel cells undergo changes in their expression markers during the maturation process. Although CK8, CK18 and CK20 are used as Merkel cell markers, CK8 is expressed in the undifferentiated state, CK18 is expressed in activated Merkel cells, and CK20 is expressed in the mature state (PLoS Genet (2016) Jul 14; 12(7):e1006151.). Merkel cells can be activated by application of the Merkel cell activating agent of the present invention. Specifically, application of a Merkel cell activating agent makes it possible to increase expression of proteins associated with synaptic vesicles, such as Piccolo, in the activated Merkel cells. Activation of Merkel cells accelerates release of neurotransmitters into synapses, thereby enhancing tactile sensation.

Another aspect of the invention relates to a synaptic vesicle enhancing agent for Merkel cells comprising Sandalore or analog thereof. Application of a synaptic vesicle enhancing agent for Merkel cells comprising Sandalore or analog thereof increases the number of synaptic vesicles in the Merkel cells. By enhancing synaptic vesicles, the efficiency of neurotransmission between Merkel cells and neurons is increased, and neurons associated with tactile sensation are activated. Activation of neurons in the dermis layer increases collagen production by dermal fibroblasts. The present invention therefore relates to a collagen production accelerating agent that includes Sandalore or its analog. Another aspect of the invention may relate to a composition comprising Sandalore or its analog, which is for treatment, prevention or alleviation of at least one symptom selected from the group consisting of itching, skin pruritus, alloknesis and allodynia.

Yet another aspect of the invention relates to a method for activating Merkel cells *in vitro* or *in vivo* and to a method for accelerating release of neurotransmitters from Merkel cells either *in vitro* or *in vivo,* comprising application of Sandalore or analog thereof. The method for activating Merkel cells *in vitro* or the method for accelerating release of neurotransmitters from Merkel cells *in vitro* comprises a step of culturing a cell group or tissue containing Merkel cells in a solution that contains Sandalore or its analog. The method for activating Merkel cells *in vivo* or the method for accelerating release of neurotransmitters from Merkel cells *in vivo* includes a step of administering Sandalore or its analog to any subject. Activation of Merkel cells *in vivo* allows treatment, prevention or alleviation of at least one symptom selected from the group consisting of itching, skin pruritus, alloknesis and allodynia. Therefore, a Merkel cell activating agent or neurotransmitter release accelerating agent of the invention may be an agent for treatment, prevention or alleviation of at least one symptom selected from the group consisting of itching, skin pruritus, alloknesis and allodynia. The subject that is administered the Sandalore or its analog may be a subject with decreased Merkel cell activity, a subject with dulled tactile sensation, a subject with decreased collagen production, or a subject suffering from or desiring prevention or alleviation of at least one symptom selected from the group consisting of itching, skin pruritus, alloknesis and allodynia. Administration of Sandalore or its analog may be administration by any route such as a transdermal, oral, transmucosal, nasal, intravenous, intraarterial or subcutaneous route, but transdermal or transmucosal administration is preferred from the viewpoint of acting on Merkel cells. When the Sandalore or its analog is to be administered percutaneously, the Sandalore or its analog may be applied to any part of the skin, such as skin of the face, head, neck, extremities or trunk.

The Merkel cell activating agent, synaptic vesicle enhancing agent and/or neurotransmitter release accelerating agent of the invention may each be incorporated in a cosmetic, drug or quasi drug. Such a drug may be administered orally or parenterally, such as by transdermal administration. For transdermal administration, the dosage form may be an external preparation for skin. An external preparation for skin is not particularly restricted so long as it can be applied to skin, and it may be applied in the form of a solution, emulsion, solid, semi-solid, powder, powder dispersion, separated water-oil bilayer, separated water-oil-powder trilayer, unguent, gel, aerosol, mousse or stick, for example. For preparation as an external preparation for skin, bases commonly used for external preparations for skin, as well as excipients, such as preservatives, emulsifying agents and pH adjustors, may also be used. For addition to a cosmetic, it may be added to a cosmetic for the face or body such as a cosmetic water, latex, essence, cream, lotion, pack, essence or gel, a makeup cosmetic such as a foundation, makeup base or concealer, or a bath preparation. Using a cosmetic containing the component of the invention activates Merkel cells and accelerates activation of neurons and associated collagen production. It may therefore be used in a cosmetic for improving wrinkles and sagging by accelerating collagen production.

The Merkel cell activating agent, synaptic vesicle enhancing agent and/or neurotransmitter release accelerating agent of the invention may be arbitrarily selected from the viewpoint of exhibiting a desired effect such as a Merkel cell activating effect, a synapse enhancing effect or an effect of accelerating release of neurotransmitters from Merkel cells, or from the viewpoint of accelerating collagen production. From the viewpoint of addition as an external preparation for skin, the Sandalore or analog thereof may be added at 0.0005% to 0.5%. From the viewpoint of exhibiting a satisfactory effect, it is preferably added at 0.001% or greater and more preferably 0.005% or greater. From the viewpoint of avoiding strong odor, it is preferably added at 0.1% or lower and more preferably 0.05% or lower.

The invention also relates to a method for assessing neurotransmitter release activity of Merkel cells in a skin sample. Specifically, the assessment method includes the following steps:
culturing the skin sample in medium containing a neurotransmitter fluorescent tracer;
applying depolarizing stimulus; and
measuring the change in fluorescent brightness value of the Merkel cells before and after depolarizing stimulus, under a fluorescent microscope. A washing step or medium exchange step may also be carried out after the step of culturing the skin sample in medium containing a fluorescent tracer. Such a method may be carried out under perfusion conditions. The method allows direct and immediate evaluation of the neurotransmitter release activity of Merkel cells.

Merkel cells are found in the maximum depth of the epidermis and perform a function as mechanically stimulated receptors. When Merkel cells are excited by mechanical stimulation and become depolarized, neurotransmitters are secreted into the synaptic cleft and a signal is transmitted through the synapse to sensory nerves. It has been suggested that Merkel cell Piezo2 channels are involved in sensing of mechanical stimulation. Merkel cells undergo changes in their expression markers during the maturation process. Merkel cells in a skin sample can be identified by using a fluorescent dye that is specifically taken up into Merkel cells, such as Quinacrine or FM dye, as the marker. According to a different aspect, a protein expressed by Merkel cells may be detected as the marker, such as one or more cell surface markers selected from the group consisting of CK8, CK18 and CK20.

A skin sample may be any sample containing Merkel cells, and non-Merkel cells may also be included. The skin sample may be derived from any animal, but is preferably derived from a human from the viewpoint of developing a cosmetic or drug. Cells other than Merkel cells may be skin cells such as epidermal cells or dermal cells, and nervous system cells such as neurons may also be included. The skin sample may be a skin sample harvested from a subject, or it may be a cultured product. More preferably, epidermal cells or dermal cells are cultured to construct a three-dimensional cultured skin model.

Neurotransmitter release activity is determined based on the amount of neurotransmitter released from the Merkel cells. When Merkel cells receive a depolarizing stimulus, they release neurotransmitters into the synaptic cleft, propagating the stimulation signal. Some of the neurotransmitters released into the synaptic cleft cause excitation of nerves as they bind to nerve receptors. Some of the neurotransmitters released into the synaptic cleft are taken back up through receptors expressed on the Merkel cells. Monoamine neurotransmitters are one type of neurotransmitter released by Merkel cells. According to the invention, neurotransmitter release activity is determined by incorporating a neurotransmitter fluorescent tracer into the Merkel cells and measuring the change in fluorescent tracer in the cells during depolarization. The neurotransmitter fluorescent tracer is dissolved in the medium to 1 to 50 µM concentration, and taken up into the Merkel cells by culturing for 30 minutes to 12 hours. The fluorescent tracer is an analog of the neurotransmitter that has observable fluorescence. From the viewpoint of observing uptake into the Merkel cells and release by the Merkel cells, FFN206 (4-(2-aminoethyl)-7-(methylamino)-2H-1-benzopyran-2-one dihydrochloride) (NPL 4:Neuron (2018)19:100(6):1401-1413) or FFN511 ((9-(2-aminoethyl)-2,3,6,7-tetrahydro-1H, 5H, 11H-[1]-benzopyrano[6,7,8-ij]quinolysine-11-one)) may be used, for example.

The fluorescent tracer in the cells during depolarization is measured by observing the sample under a fluorescent microscope, and measuring the fluorescent brightness value before depolarization and the fluorescent brightness value after the depolarizing stimulus. The fluorescent brightness value measurement may also be carried out periodically. In the case of periodic measurement, the fluorescence will undergo gradual discoloration, and therefore the fluorescent brightness value may be corrected in consideration of discoloration. After the depolarizing stimulus, the fluorescent tracer is released into the synaptic cleft together with the neurotransmitter, thereby decreasing the fluorescent brightness value in the cells. The fluorescent brightness value may be measured in the whole cell, or it may be determined in a specific region near the synapse, such as in a synaptic vesicle region.

The depolarizing stimulus may be mechanical stimulation or drug stimulation. Mechanical stimulation may be applied using a probe on the Merkel cells. The mechanical stimulation may also be applied until deformation of the cells is observed. Drug stimulation may be applied using a drug that causes depolarization. Sandalore and its analogs are examples of such drugs. More specifically, depolarizing stimulus may be applied by introducing a drug-containing medium that causes depolarization under perfusion conditions.

Another aspect of the invention relates to a method for screening a neurotransmitter release accelerating agent for Merkel cells, utilizing a method for assessing neurotransmitter release activity of Merkel cells. Such a screening method includes the following steps, specifically:
culturing the skin sample in medium containing a neurotransmitter fluorescent tracer;
adding a candidate substance for a neurotransmitter release accelerating agent; and
measuring the change in fluorescent brightness value of the Merkel cells before and after addition, under a fluorescent microscope. The screening method allows screening of a neurotransmitter release accelerating agent that accelerates neurotransmitter release in Merkel cells. A washing step or medium exchange step may also be carried out after the step of culturing the skin sample in medium containing a fluorescent tracer. Such a method may be carried out under perfusion conditions. When the fluorescent brightness value has significantly decreased, the candidate substance may be selected as a neurotransmitter release accelerating agent for Merkel cells.

Candidate substances for neurotransmitter release accelerating agents include substances belonging to a library such as a cosmetic material library, extract library, drug library or compound library. A neurotransmitter release accelerating agent can induce depolarization of Merkel cells, and may therefore be considered to be a depolarizing stimulator or Merkel cell activating agent.

The neurotransmitter release accelerating agent induces depolarization of Merkel cells and accelerates neurotransmitter release from the Merkel cells. By accelerating neurotransmitter release, the efficiency of neurotransmission between Merkel cells and neurons is increased, and neurons associated with tactile sensation are activated. According to another aspect, a lower concentration of neurotransmitter release accelerating agent can be used to sensitize Merkel cells, i.e. to convert the Merkel cells to a more easily depolarizable state, so that it may also be considered to be a Merkel cell sensitization accelerating agent. The Merkel cells are therefore activated even by weak mechanical stimulation. The efficiency of neurotransmission between Merkel cells and neurons is thereby increased, and neurons associated with tactile sensation are activated. Activation of neurons in the dermis layer increases collagen production by dermal fibroblasts. The invention therefore also relates to a collagen production accelerating agent that includes a neurotransmitter release accelerating agent.

Yet another aspect of the invention relates to a neurotransmitter release accelerating agent selected by the screening method described above. More specifically, Sandalore and its analogs are screened as neurotransmitter release accelerating agents. The invention therefore further relates to a neurotransmitter release accelerating agent or collagen production accelerating agent including one or more selected from the group consisting of Sandalore and its analogs.

### EXAMPLES

### Example 1: Distribution of Merkel cells in scalp sample and abdominal skin sample

### Skin sample

Human scalp from the temporal and occipital regions or abdominal skin was taken from healthy subjects (20 to 60 years of age: total of 13) that had received common cosmetic surgery or plastic surgery, and used after obtaining informed consent and ethical approval (Muenster University). The human scalp skin was taken from three males and three females, while the abdominal skin was taken from 6 females and one male.

### Fluorescent immunohistochemical staining

Each of the scalp samples and abdominal skin samples were cut into 4 mm pieces, embedded in OCT and frozen in liquid nitrogen. The OCT-embedded samples were sectioned using a Leica cryostat. As the primary antibody staining, the tissue frozen section was fixed with 4% paraformaldehyde, preincubated with 10% goat serum (for OR2AT4) or 5% goat serum + 0.3% TritonX-100, and then incubated overnight at 4°C in the corresponding primary antibody solution. Each was then incubated for 45 minutes at room temperature in the secondary antibody solution. DAPI (1 µg/ml) was used for nuclear staining.

The primary antibodies used were anti-OR2AT4 antibody (distributor: Eurogentech, Product No.: custom made, 1910432), anti-CK8 antibody (distributor: Progen, Product No.: GP-CK8) and anti-CK18 antibody (distributor: Progen, Product No.: GP-CK18), and the subsequent secondary antibodies used were Goat anti-Rabbit IgG (H+L) Cross-Adsorbed Secondary Antibody (distributor: Invitrogen, Product No.: A-11008), Goat anti-Mouse IgG (H+L) Highly Cross-Adsorbed Secondary Antibody (distributor: Invitrogen, Product No.: A-11030) and Fluorescein (FITC)- conjugated AffiniPure Goat Anti Guinea Pig IgG (H+L) (distributor: Jackson ImmunoResearch, Product No.: 106-095-003), in combination with each of the primary antibodies, respectively. The nuclear stain used was DAPI (distributor: Sigma-Aldrich, Product No.: 10236276001).

The co-staining results with OR2AT4 and CK8 were represented (Fig. 1A: scalp, Fig. 1B: abdominal skin), the number of cells double-positive for OR2AT4 and CK8 in a specified region were counted (Fig. 1C left, Fig. 1D left), and the ratio of the number of cells double-positive for OR2AT4 and CK8 with respect to the number of CK8-positive cells was represented (Fig. 1C right, Fig. 1D right). In the scalp and abdominal skin epidermis, approximately 50 to 60% of the CK8-positive immature Merkel cells expressed OR2AT4.

The co-staining results with OR2AT4 and CK18 were represented (Fig. 2A: scalp, Fig. 2B: abdominal skin), the number of cells double-positive for OR2AT4 and CK18 in a specified region were counted (Fig. 2C left, Fig. 2D left), and the ratio of the number of cells double-positive for OR2AT4 and CK18 with respect to the number of CK18-positive cells was represented (Fig. 2C right, Fig. 2D right). In the scalp and abdominal skin epidermis, approximately 50% of the CK18-positive intermediate-mature Merkel cells expressed OR2AT4.

The co-staining results with OR2AT4 and CK20 were represented (Fig. 3A: scalp, Fig. 3B: abdominal skin), the number of cells double-positive for OR2AT4 and CK20 in a specified region were counted (Fig. 3C left, Fig. 3D left), and the ratio of the number of cells double-positive for OR2AT4 and CK8 with respect to the number of CK20-positive cells was represented (Fig. 3C right, Fig. 3D right).
In the scalp and abdominal skin epidermis, approximately 70 to 85% of the CK20-positive mature Merkel cells expressed OR2AT4.

### Quantitative immunohistomorphology measurement

The staining intensity was evaluated for a clearly defined reference region by quantitative (immune) histomorphological measurement 31, 32 using NIH ImageJ software (NIH, Bethesda, MD, USA).

### Example 2: Change in Piccolo expression by application of Sandalore onto scalp sample

### Skin sample

Human scalp from the temporal and occipital regions was taken from healthy female subjects (47 to 60 years of age) that had received common cosmetic surgery, and used after obtaining informed consent and ethical approval (Muenster University).

### Fluorescent immunohistochemical staining

Scalp samples including hair follicles were taken using a 6 mm punch from the occipital region (donors 1, 2, 3 and 4) or the temporal region (donor 5) of female subjects: a 60-year-old female (donor 1), a 55-year-old male (donor 2), a 47-year-old male (donor 3), a 40-year-old female (donor 4) and a 40-year old female donor 5). The obtained samples were placed in 25 ng/ml NGF-added DMEM/F12 medium and cultured in a 5% CO₂ humidified atmosphere at 37°C (day 0). On day 1, the medium was exchanged with medium containing Sandalore (500 µM). A 0.1% DMSO-containing medium was used as a control. On day 3 the samples were collected, embedded in OCT and frozen in liquid nitrogen. The OCT-embedded samples were sectioned using a Leica cryostat. As the primary antibody staining, the tissue frozen section was fixed with 4% paraformaldehyde, preincubated with 5% goat serum + 0.3% TritonX-100, and then incubated overnight at 4°C in the corresponding primary antibody solution. Each was then incubated for 45 minutes at room temperature in the secondary antibody solution. DAPI (1 µg/ml) was used for nuclear staining.

The primary antibodies used were anti-CK20 antibody (distributor: Progen, Product No.: Product No.: 61054) and anti-Piccolo antibody (distributor: Novus Biologicals (Tocris), Product No.: NBP1-90250), and the subsequent secondary antibodies used were Goat anti-Rabbit IgG (H+L) Cross-Adsorbed Secondary Antibody (distributor: Invitrogen, Product No.: A-11008) and Goat anti-Mouse IgG (H+L) Highly Cross-Adsorbed Secondary Antibody (distributor: Invitrogen, Product No.: A-11030), in combination with each of the primary antibodies, respectively. The nuclear stain used was DAPI (distributor: Sigma-Aldrich, Product No.: 10236276001). Fluorescent microscope photographs are shown in Fig. 4 (Fig. 4A: Vehicle (control), Fig. 4B: Sandalore). The number of CK20-positive cells expressing Piccolo in a specified region in a photograph taken under a fluorescent microscope were counted and shown in a graph (Fig. 4C). Sandalore increased the number of Merkel cells expressing Piccolo.

### Example 3: Accelerating effect of sensory neurons on type I collagen production by fibroblasts

### (1) Culturing of sensory neurons

Human iPS cell-derived sensory neuron progenitor cells (i-SNs, ax0055, Axol Bioscience, Cambridge, United Kingdom) were cultured according to the manufacturer's instructions (Fig. 5A). Specifically, the cells were plated in a 24-well plate coated with Sure-Bond XF (ax0053) and cultured in Sensory Neuron Maintenance Medium (SNMM, ax0060) with addition of 25 ng/mL GDNF, 25 ng/mL BDNF, 10 ng/mL NGF and 10 ng/mL NT-3. Culturing was continued for 5 weeks while exchanging half of the medium every 3 to 4 days, resulting in differentiation to sensory neurons. After culturing for 5 weeks, the culture solution was collected and the supernatant was obtained.

### (2) Type I collagen production by fibroblasts stimulated with sensory neurons

### (2-1) Stimulation by sensory neurons

The fibroblasts were obtained by isolating neonatal foreskin fibroblasts after informed consent, and growing them for 2 days in a humidified atmosphere of 95% air and 5% CO₂ at 37°C, in Dulbecco's Modified Eagle medium (Life Technologies Japan Ltd., Tokyo, Japan) containing 10% fetal bovine serum. The supernatant (300 µl) of the culture solution obtained in method (1) was mixed with the fibroblast culture solution in a proportion of 1:1 and cultured for 4 hours, after which the collagen production was measured by the method of (2-2) below. The control used was a mixture of the medium for the iPS cell-derived sensory neuron progenitor cells, instead of the culture solution supernatant, with the fibroblast culture solution in a proportion of 1:1.

### (2-2) Measurement of type I collagen production by quantitative real-time PCR.

After serum starvation, total RNA was isolated from the fibroblasts cultured for 4 hours in the presence of or in the absence of neuron medium supernatant, using an RNeasy Mini Kit (250) (Qiagen #74106). The mRNA expression level of type I collagen was measured by quantitative real-time PCR. Specifically, the measurement was carried out using TaqMan RNA-to-C 1-Step Kit (Applied Biosystems #4392938), TaqMan probe and type I collagen α1 primer (Hs00164004, Taqman, Applied Biosystems). The internal control used was β-actin (Hs01060665), and the expression level was normalized against β-actin.

The results are shown in Fig. 5B. When the supernatant was added to the neuron culture solution (conditioned medium), mRNA expression level of type I collagen by fibroblasts was significantly increased compared to the non-added control. This suggests that a neuron-derived component acts to accelerate production of collagen by fibroblasts.

### Example 4: Effect of accelerating collagen production via neural activation, by stimulation with different substances

In order to confirm that type I collagen production by fibroblasts was accelerated by neural activation, a culture supernatant of neurons stimulated by lavender oil, which was confirmed to activate nerves, was used and type I collagen production was measured.

### (1) Test substances

Lavender oil purchased from Vaillant, France (essential oil containing ≥30% linalyl acetate obtained by steam distillation of *Lavandula vera* DC. flowers) was used. Also used as test substances were the publicly known TRPV1 ligands capsaicin (Cap) (Sigma-Aldrich Japan, KK.) and cinnamaldehyde (CNM 125 µM) (Sigma-Aldrich Japan, KK.).

### (2) Culturing of human iPS cell-derived sensory neuron progenitor cells

The total amount (600 µl) of the supernatant of neural progenitor cells cultured for at least 5 weeks using the same materials and method as Example 4 was collected, and then 500 µl of medium, prepared by adding the test substance of (1) to fibroblast-containing medium for a final lavender oil concentration of 0.005%, was added to the neural progenitor cells and the mixture was allowed to stand for 15 minutes in an incubator. The same test was carried out using 0.25 µM capsaicin (Cap 0.25 µM) and cinnamaldehyde (CNM 125 µM) as test substances. The supernatant containing the test substance was then collected and DMEM was added to prepare a mixture with a composition of supernatant:DMEM = 1:3. The mixture was applied in an amount of 1 ml each to fibroblasts cultured in a 12-well plate by the same method as Example 4, and after standing for 4 hours, the RNA was collected. As a control, DMEM prepared with the test substance at the same final lavender oil concentration of 0.005% was directly mixed with fibroblasts and allowed to stand for 4 hours, after which the RNA was collected. The collected RNA was measured for type I collagen by quantitative real-time PCR in the same manner as Example 4.

The results are shown in Fig. 6. As shown in Fig. 6A, the culture supernatants of iPS cell-derived sensory neuron progenitor cells stimulated with lavender oil had accelerated type I collagen production by the fibroblasts. However, as shown in Fig. 6B, direct addition of lavender oil in the same concentration without neural activation to the fibroblasts did not show any significant acceleration of type I collagen production.

### Example 5: Effect of direct addition of high-concentration test substance

The effect of directly adding the test substance at higher concentration than in Example 5 was examined. Specifically, collagen was measured after direct application to fibroblasts by the same method as the control experiment of Example 5, except that the test substance of Example 5 was added to a final lavender oil concentration of 0.02%.

The results are shown in Fig. 7. As shown in Fig. 7, acceleration of type I collagen production by fibroblasts was observed when the test substance was at high concentration. However, the fact that such an effect was not seen at low concentration (Fig. 6B) suggests that type I collagen production by fibroblasts was accelerated via neural activation by neural activation substances such as lavender oil.

### Example 6: Release of neurotransmitters by Merkel cells in fresh human skin (ex vivo)

### Sample preparation

Fresh skin excised from the abdominal region of a 42-year-old female healthy subject and a 48-year-old healthy subject, both having undergone cosmetic surgery, was used for experimentation within 5 days, after obtaining informed consent and ethical approval. The skin was exposed to 25 units/ml of a dispase-PBS solution for 15 minutes at 37°C to detach the epidermis.

The epidermis isolated from the fresh human skin was cultured for 30 minutes in a high-potassium extracellular solution (5 mM NaCl, 140 mM KCl, 10 mM HEPES (pH 7.4), 10 mM D-glucose, 2 mM MgCl₂ and 2 mM CaCl₂) containing FFN206 (Tocris, Catalog No. 5043), and the fluorescent-labeled neurotransmitter FFN206 was specifically taken up into the Merkel cells.

The FFN206 signal in the epidermis containing the Merkel cells that had taken up FFN206 was observed for 10 minutes under circulating conditions. BSS (Balanced salt solution) solution (140 mM NaCl, 5 mM KCl, 10 mM HEPES (pH 7.4), 10 mM D-glucose, 2 mM MgCl₂ and 2 mM CaCl₂) was circulated for the first 5 minutes, as baseline. After 5 minutes (300 s) until the end of observation (600 s), circulation was with 500 µM Sandalore (Givaudan)/BSS solution (A). As a control, BSS solution alone was circulated from 5 minutes (300 s) until the end of observation (600 s). The brightness value data were acquired using a Fluoview (Olympus).

The cell response was evaluated based on the brightness value of FFN206, using cellSens (Olympus) and MATLAB (Mathworks). An ROI was created following the FFN206 signal, evaluating the time-dependent change in average brightness value within the ROI. The change in brightness value of the baseline in each ROI was determined by nonlinear approximation, and the brightness value with the effect of discoloration eliminated was determined based on the approximation results for baseline. The average brightness value at baseline 300 s was divided by the brightness value at each time point to calculate the normalized brightness value (%). The index for evaluation of cellular response was lowering of the normalized brightness value by at least 3 S.D. from the average value for baseline in each ROI during Sandalore stimulation (after 300 s) (Fig. 8A). In the control, no lowering was observed in the brightness value by BSS solution circulation after 5 minutes (Fig. 8B).

In order to confirm release of FFN206 by stimulation with 500 µM Sandalore, live cell imaging was carried out for 10 minutes and the brightness value of the FFN206 signal was observed. The results showed a response by Merkel cells to Sandalore stimulation (Fig. 8 and Fig. 9). In Fig. 8 and Fig. 9 it is seen that the brightness value continued to decrease beyond the reaction line calculated from baseline, after inflow of Sandalore stimulation at 300 s. Fig. 8(A) shows change in brightness value in mouse epidermis, with Sandalore activating Merkel cells via OR2AT4 expressed in the Merkel cells, and acceleration of neurotransmitter release being observed.

## Claims

1. A Merkel cell activating agent, comprising a compound represented by the following formula: 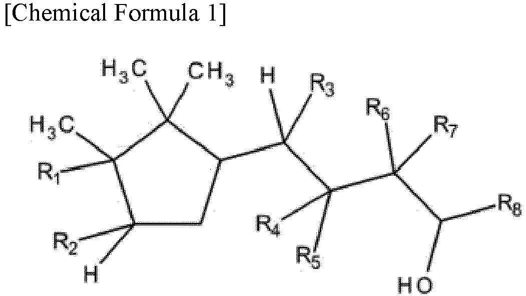 [wherein
R₁ and R₂ together form a double bond, or R₁ and R₂ together form a cyclopropyl group;
R₃ and R₄ are the same or different and are independently selected from hydrogen and methyl, or R₃ and R₄ together form a double bond;
R₅ and R₆ are the same or different and are independently selected from hydrogen and methyl, or R₅ and R₆ together form a double bond; or R₅ and R₆ together form a cyclopropyl group;
R₇ is methyl or ethyl; and
Rg is hydrogen or methyl].

2. The activating agent according to claim 1, wherein the compound is Sandalore or Brahmanol.

3. The activating agent according to claim 1 or 2, which enhances tactile sensation.

4. A synaptic vesicle enhancing agent for Merkel cells, comprising a compound represented by the following formula: 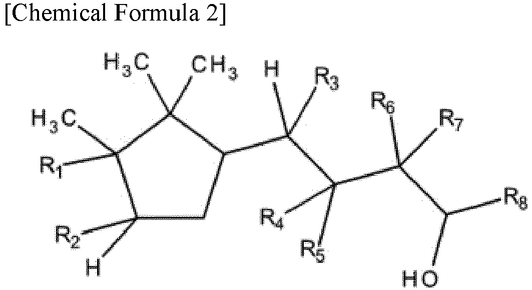 [wherein
R₁ and R₂ together form a double bond, or R₁ and R₂ together form a cyclopropyl group;
R₃ and R₄ are the same or different and are independently selected from hydrogen and methyl, or R₃ and R₄ together form a double bond;
R₅ and R₆ are the same or different and are independently selected from hydrogen and methyl, or R₅ and R₆ together form a double bond; or R₅ and R₆ together form a cyclopropyl group;
R₇ is methyl or ethyl; and
Rg is hydrogen or methyl].

5. The enhancing agent according to claim 4, wherein the compound is Sandalore or Brahmanol.

6. The enhancing agent according to claim 4 or 5, wherein the enhancing agent increases the efficiency of neurotransmission between Merkel cells and neurons.

7. The enhancing agent according to any one of claims 4 to 6, which enhances tactile sensation.

8. The enhancing agent according to any one of claims 4 to 7, which enhances collagen production.

9. A method for assessing neurotransmitter release activity of Merkel cells in a skin sample, comprising:
culturing the skin sample in medium containing a neurotransmitter fluorescent tracer;
applying depolarizing stimulus; and
measuring the change in fluorescent brightness value of the Merkel cells before and after depolarizing stimulus, under a fluorescent microscope.

10. The assessment method according to claim 9, wherein the neurotransmitter fluorescent tracer is a monoamine neurotransmitter fluorescent tracer.

11. The assessment method according to claim 9 or 10, wherein the skin sample is a human skin sample.

12. A method for screening a neurotransmitter release accelerating agent for Merkel cells in a skin sample, comprising:
culturing the skin sample in medium containing a neurotransmitter fluorescent tracer;
adding a candidate substance for a neurotransmitter release accelerating agent; and
measuring the change in fluorescent brightness value of the Merkel cells before and after addition, under a fluorescent microscope.

13. The screening method according to claim 12, wherein the neurotransmitter fluorescent tracer is a monoamine neurotransmitter fluorescent tracer.

14. The screening method according to claim 12 or 13, wherein the skin sample is a human skin sample.

15. A neurotransmitter release accelerating agent for Merkel cells, comprising a compound represented by the following formula: 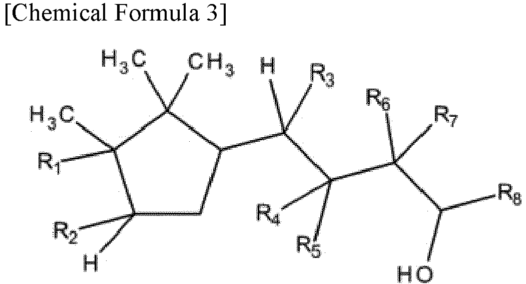 [wherein
R₁ and R₂ together form a double bond, or R₁ and R₂ together form a cyclopropyl group;
R₃ and R₄ are the same or different and are independently selected from hydrogen and methyl, or R₃ and R₄ together form a double bond;
R₅ and R₆ are the same or different and are independently selected from hydrogen and methyl, or R₅ and R₆ together form a double bond; or R₅ and R₆ together form a cyclopropyl group;
R₇ is methyl or ethyl; and
Rg is hydrogen or methyl].

16. The neurotransmitter release accelerating agent according to claim 15, wherein the compound is Sandalore or Brahmanol.

17. The neurotransmitter release accelerating agent according to claim 15 or 16, which enhances tactile sensation.

18. The neurotransmitter release accelerating agent according to any one of claims 15 to 17, which enhances collagen production.
